# EUROPÄISCHE PATENTSCHRIFT

(11) **EP 1 348 388 B1**
(45) Veröffentlichungstag und Bekanntmachung des Hinweises auf die Patenterteilung: **14.06.2006**
(21) Anmeldenummer: 03005812.7
(22) Anmeldetag: 14.03.2003
(51) Int. Cl.: A61B 17/70

(54) **Knochenverankerungsvorrichtung zum Stabilisieren von Knochensegmenten**
Bone anchoring device for stabilisation of bone segments
Dispositif d'ancrage d'os pour stabiliser des segments osseux

(30) Priorität: 27.03.2002 DE 10213855
(43) Veröffentlichungstag der Anmeldung: 01.10.2003
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE)
(74) Vertreter: Hofer, Dorothea

(56) Entgegenhaltungen:
- FR-A- 2 624 720
- FR-A- 2 796 545
- US-A- 5 554 157
- US-A- 5 690 630
- US-A- 5 728 098
- US-A1- 2003 004 511

## Beschreibung

Die Erfindung betrifft eine Knochenverankerungsvorrichtung zum Stabilisieren von Knochensegmenten mit einem Knochenverankerungselement und einem Aufnahmeteil zum gelenkigen Verbinden des Knochenverankerungselementes mit einem Stab und ein Aufnahmeteil einer derartigen Knochenverankerungsvorrichtung nach den Oberbegriffen der Patentansprüche 1 und 2.

Aus der DE 37 11 013 C1 ist eine Pedikelschraube zur Stabilisierung von Wirbelsäulensegmenten bekannt, bei der das Schraubenelement gelenkig mit einem Aufnahmeteil verbunden ist. Das Aufnahmeteil umfaßt zwei den Kopf des Schraubenelements umschließende Hälften und einen diese zusammenhaltenden Haltering. Ferner weist das Aufnahmeteil Aufnahmeschlitze zum Einlegen einer Gewindestange auf. Eine Blockierung des Kopfes in dem Aufnahmeteil und eine Fixierung der Gewindestange erfolgt durch auf die Gewindestange zu beiden Seiten des Aufnahmeteils aufgeschraubte mutternförmige Fixierschrauben, die seitlich am Aufnahmeteil angreifen.

Eine Knochenverankerungsvorrichtung nach dem Oberbegriff des Patentanspruches 2 und ein Aufnahmeteil nach dem Oberbegriff des Patentanspruches 1 ist aus der DE 39 23 996 C2 bekannt. Bei der bekannten Vorrichtung ist zum Fixieren der Winkelstellung der Knochenschraube relativ zu dem Aufnahmeteil ein auf den Kopf der Knochenschraube einwirkendes Druckelement vorgesehen. Die Fixierung des Stabes, der als Gewindestab ausgebildet ist, erfolgt durch auf den Stab aufgeschraubte Muttern, die seitlich am Aufnahmeteil angreifen. In zusammengesetztem Zustand wird über das Druckelement in axialer Richtung des Aufnahmeteils eine Kraft so auf dem Kopf ausgeübt, daß dieser im Aufnahmeteil blockiert wird.

Aus der DE 43 07 576 C1 ist eine Knochenverankerungsvorrichtung nach dem Oberbegriff des Patentanspruches 2 und ein Aufnahmeteil nach dem Oberbegriff des Patentanspruches 1 bekannt, bei der ebenfalls ein Druckelement zur Fixierung des Kopfes vorhanden ist. Eine Fixierung von Kopf und Stab erfolgt mittels einer in das Aufnahmeteil einschraubbaren Fixierschraube und einer auf das Aufnahmeteil aufgeschraubten Sicherungsmutter, die eine Kraft in axialer Richtung des Aufnahmeteils auf das Druckelement ausüben.

In der US 5,728,098 sind eine Knochenverankerungsvorrichtung nach dem Oberbegriff des Anspruchs 2 und ein Aufnahmeteil nach dem Oberbegriff des Anspruchs 1 beschrieben. Eine Fixierung von Kopf und Stab erfolgen getrennt über zwei Ringe aus Memorymetall. Dazu weist das Aufnahmeteil ein Paar einander gegenüberliegender oberer Schlitze und ein Paar einander gegenüberliegender unterer Schlitze auf. Aufnahmeteil, Schraube und unterer Ring werden lose vormontiert und die Knochenverankerungsvorrichtung in diesem Zustand in den Knochen eingeschraubt. Anschließend wird der Stab eingelegt und der obere Ring montiert. Durch die Körperwärme ziehen sich die beiden Ringe aus Memorymetall zusammen, so dass Kopf und Stab getrennt voneinander durch den jeweiligen Ring in dem Aufnahmeteil fixiert werden.

Es ist Aufgabe der Erfindung, eine verbesserte Knochenverankerungsvorrichtung bzw. ein Aufnahmeteil für eine solche bereitzustellen, wobei weniger Bauteile benötigt werden, die vom Anwender leichter zu handhaben sind und die von den Dimensionen her filigraner gestaltet werden können.

Die Aufgabe wird gelöst durch ein Aufnahmeteil gemäß Patentanspruch 1 und eine Knochenverankerungsvorrichtung nach Patentanspruch 2. Weiterbildungen der Erfindung sind in den Unteransprüchen angegeben.

Bei der erfindungsgemäße Knochenverankerungsvorrichtung ist durch eine Kombination von seitlich und in axialer Richtung des Aufnahmeteils wirkenden Druckkräften eine äußerst sichere Verklemmung gegeben.

Weitere Merkmale und Zweckmäßigkeiten der Erfindung ergeben sich aus der Beschreibung von Ausführungsbeispielen anhand der Figuren.

Von den Figuren zeigen:
- Fig. 1: eine Knochenverankerungsvorrichtung nach einer ersten Ausführungsform im zusammengesetzten Zustand in teilgeschnittener Darstellung;
- Fig. 2: die Knochenverankerungsvorrichtung von Fig. 1 in perspektivischer Explosionsdarstellung;
- Fig. 3: die Knochenverankerungsvorrichtung von Fig. 1 in teilgeschnittener Darstellung im unbelasteten Zustand;
- Fig. 4: die Knochenverankerungsvorrichtung von Fig. 1 in belastetem Zustand;
- Fig. 5: das Detail I von Fig. 4 in vergrößerter schematisierter Darstellung;
- Fig. 6: eine abgewandelte Ausführungsform der Knochenverankerungsvorrichtung von Fig. 1 in teilgeschnittener Darstellung;
- Fig. 7: eine vergrößerte perspektivische Schnittdarstellung eines Elements der Knochenverankerungsvorrichtung gemäß Fig. 6;
- Fig. 8: eine zweite Ausführungsform der Knochenverankerungsvorrichtung; und
- Fig. 9: eine vergrößerte Darstellung eines Details II einer Abwandlung der Ausführungsform von Fig. 7.

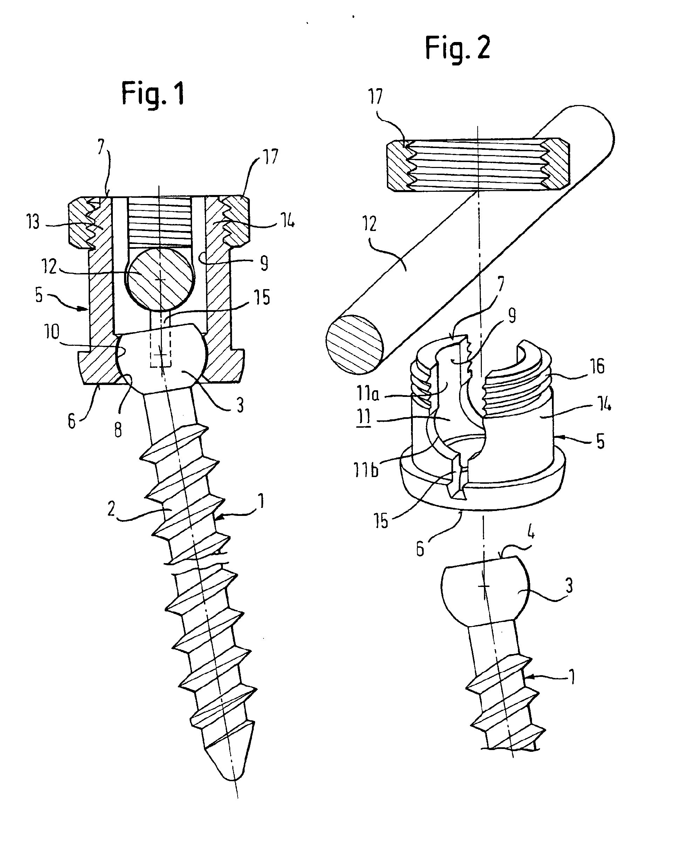

Wie aus den Figuren 1 bis 4 ersichtlich ist, weist die Knochenverankerungsvorrichtung in einer ersten Ausführungsform ein Schraubenelement 1 mit einem Gewindeabschnitt 2 mit einem Knochengewinde und einem Kopf 3 auf. Der Kopf 3 ist angrenzend an den Gewindeabschnitt kugelsegmentförmig ausgebildet. Koaxial zur Gewindeachse und auf dem Gewindeabschnitt gegenüberliegenden Ende weist der Kopf 3 eine Ausnehmung 4 zum Ineingriffbringen mit einem Schraubwerkzeug auf.

Die Knochenverankerungsvorrichtung beinhaltet ferner ein im wesentlichen zylindrisch ausgebildetes Aufnahmeteil 5. Das Aufnahmeteil weist ein erstes Ende 6 und ein diesem gegenüberliegendes zweites Ende 7 auf. Angrenzend an das erste Ende 6 ist eine axialsymmetrisch ausgerichtete Bohrung 8 vorgesehen, deren Durchmesser größer als der des Gewindeabschnittes 2 und kleiner als der des Kopfes 3 ist. Das Aufnahmeteil 5 weist ferner eine koaxiale zweite Bohrung 9 auf, die auf dem der ersten Bohrung 8 gegenüberliegenden Ende 7 des Aufnahmeteiles offen ist und deren Durchmesser so groß ist, daß das Schraubenelement 1 durch das offene Ende mit seinem Gewindeabschnitt und seinem Kopf einführbar ist. Zwischen der ersten Bohrung 8 und der zweiten Bohrung 9 ist ein koaxialer Abschnitt 10 vorgesehen, der unmittelbar an die erste Bohrung 8 angrenzt und der kugelsegmentförmig ausgebildet ist mit einem Radius, der, wenn die Knochenverankerungsvorrichtung zusammengesetzt ist und fixiert ist, im wesentlichen gleich dem Radius des kugelsegmentförmigen Abschnittes des Kopfes 3 ist. Durch die kugelsegmentförmige Ausbildung ist die Bohrung 9 derart verengt, daß der Abschnitt 10 eine sphärische Fassung für den Kopf 3 bildet.

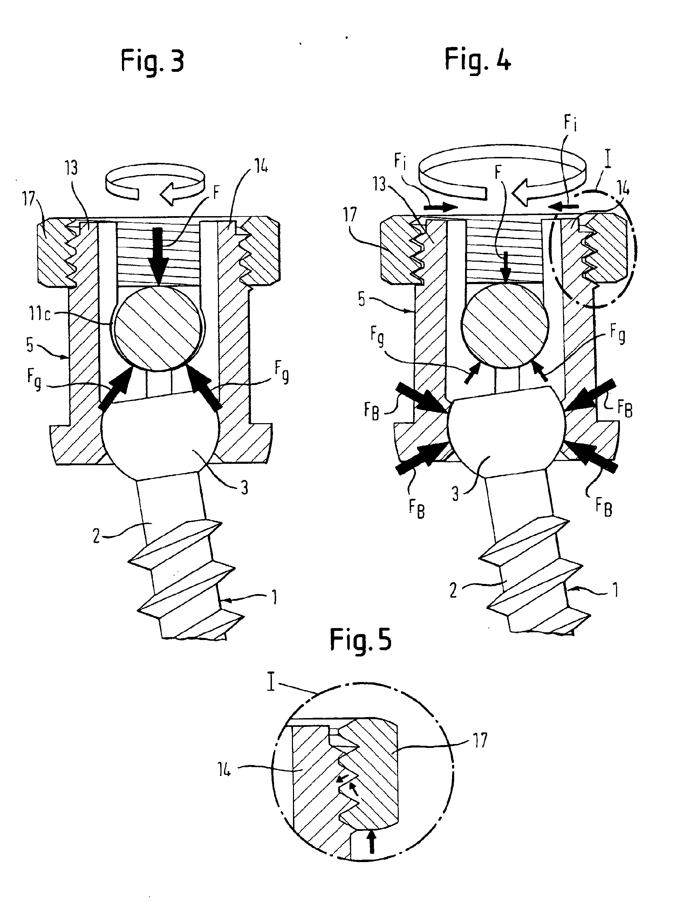

Das Aufnahmeteil 5 weist ferner eine sich von dem zweiten Ende 7 in Richtung des ersten Endes erstreckende Ausnehmung 11 auf, die im wesentlichen U-förmig ist und einen Kanal bildet, in den ein Stab 12 einlegbar ist. Durch die Ausnehmung 11 sind zwei freie Schenkel 13, 14 des Aufnahmeteils gebildet. Die Ausnehmung 11 ist ferner so ausgebildet, daß sie einen ersten Abschnitt 11a mit einer Breite aufweist, die gerade so bemessen ist, daß der Stab 12 ohne Verklemmen eingelegt werden kann und einen zweiten Bereich 11b, der den eigentlichen Kanal bildet, in dem der Stab 12 ruht. Der Bereich 11b beinhaltet den Grund des Kanals und ist im wesentliche sphärisch ausgebildet mit einem Durchmesser in der Breite, der größer ist als der Durchmesser des Stabes 12. Somit ist, wie in Fig. 3 gezeigt ist, zwischen der Kanalwand und dem eingelegten Stab ein kleiner Zwischenraum 11c vorhanden, so daß der eingelegte Stab 12 eine gewisse Beweglichkeit quer zu seiner Längsachse aufweist.

Ferner weist das Aufnahmeteil 5 zwei einander gegenüberliegende sich stirnseitig vom Grund des Kanals in Richtung auf das erste Ende 6 hin erstreckende schlitzförmige Ausnehmungen 15 auf. Die Ausnehmungen erstrecken sich bis zu einer vorbestimmten Tiefe in den kugelsegmentförmigen Abschnitt 10. Die Breite der schlitzförmigen Ausnehmungen 15 ist so bemessen, daß die freien Schenkel 13, 14 in vorgegebenem Maße elastisch gegeneinander bewegbar sind.

Ferner ist an dem Aufnahmeteil 5 im Bereich der freien Schenkel 13, 14 angrenzend an deren freies Ende 7 ein Abschnitt mit einem Außengewinde 16 ausgebildet. Das Außengewinde 16 ist als metrisches Gewinde ausgebildet.

Es ist eine Mutter 17 vorgesehen mit einem Innengewinde, das mit dem Außengewinde 16 zusammenwirkt. Die Länge des Außengewindeabschnitts und die Abmessungen der Mutter 17 in axialer Richtung sind so ausgebildet, daß bei eingeschraubtem Zustand die Mutter 17 auf den Stab 12 drückt.

Im Betrieb wird das Schraubenelement 1 von dem offenen Ende der zweiten Bohrung 9 her in die erste Bohrung 8 eingeführt bis der Kopf 3 mit seiner Unterseite an den oberen Rand des kugelsegmentförmigen Abschnittes 10, der die sphärische Fassung bildet, anstößt. Beim weiteren Einführen werden die Schenkel 13, 14 aufgrund ihrer durch die schlitzförmige Ausnehmung 15 verursachten Elastizität etwas auseinandergedrückt, so daß der Schraubenkopf in die sphärische Fassung 10 einschnappt. Dort wird er frei beweglich gehalten und die Schraube kann in den Knochen durch Eingreifen des Eindrehwerkzeuges in die Ausnehmung 4 eingeschraubt werden.

Anschließend wird der Stab 12 eingelegt und die Mutter 16 lose aufgeschraubt. Es erfolgt dann eine korrekte Ausrichtung des Aufnahmeteils 5 relativ zu dem Stab 12. In dieser ausgerichteten Stellung wird die Mutter 17 so fest angezogen, bis sie mit ihrem dem Stab zugewandten Ende auf den Stab 12 drückt und somit eine durch den Pfeil F in Fig. 3 gekennzeichnete Kraft auf den Stab 12 ausübt. Somit wird der Stab 12 in dem Kanal festgeklemmt. Die auf den Stab über die Mutter 17 wirkende Kraft F erzeugt Gegenkraftkomponenten F_{G} vom Grund des Kanals her, die durch die Pfeile F_{G} in Fig. 3 und 4 dargestellt sind. Wie in Fig. 5 dargestellt ist, wirkt diese Gegenkraft über den Stab auf den unteren Rand der Mutter 17, wodurch über die Gewindeflanken von Mutter und Aufnahmeteil eine nach innen gerichtete Kraftkomponente Fᵢ (wie in Fig. 4 dargestellt ist) erzeugt wird. Ein weiteres Aufschrauben der Mutter 17 führt dazu, daß diese Gegenkraftkomponenten F_{G} so groß werden, daß die Schenkel 13, 14 eine so große nach innen gerichtete Kraft erfahren, daß sie zusammengedrückt werden, was aufgrund ihrer durch die schlitzförmigen Ausnehmungen 15 hervorgerufene Elastizität möglich ist. Durch das Zusammendrücken der Schenkel 13, 14 schließt sich die sphärische Fassung 10 fest um den kugelsegmentförmigen Kopf und blockiert diesen in seiner Stellung. Dies wird in Fig. 4 durch die auf den sphärischen Kopf wirkenden diesen blockierenden radialen Kraftkomponenten F_{B} dargestellt. Wie insbesondere aus den Fig. 3 und 4 ersichtlich ist, verengt sich der in nicht belastetem Zustand der Vorrichtung ausgeweitete Kanal derart, daß der Stab 12 an der Kanalwand 11b anliegt.

Insgesamt wird somit mit einem einzigen Fixierelement in Form der Mutter 17 eine zuverlässige Fixierung von Stab und Kopf erreicht.

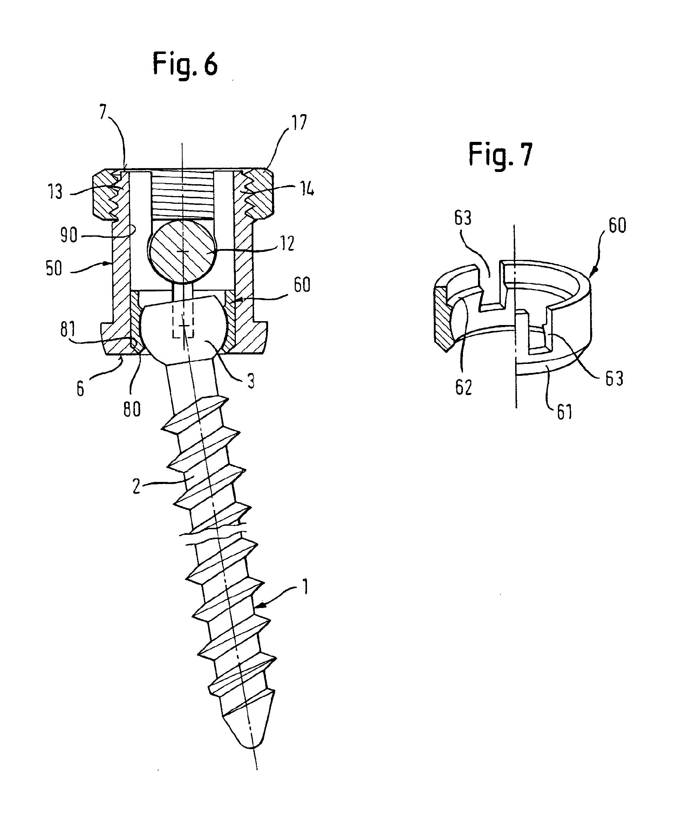

In den Fig. 6 und 7 ist eine abgewandelte Ausführungsform der Knochenverankerungsvorrichtung gemäß den Fig. 1 bis 4 gezeigt, die sich von dieser durch die Fassung des Kopfes 3 in dem Aufnahmeteil 5 unterscheidet. Gleiche Teile werden daher mit den selben Bezugszeichen beschrieben.

Das Aufnahmeteil 50 unterscheidet sich von dem Aufnahmeteil 5 dadurch, daß es angrenzend an das erste Ende eine erste Bohrung 80 aufweist, deren Durchmesser etwas größer ist als der Durchmesser des kugelsegmentförmigen Kopfes 3. Die Bohrung 80 erweitert sich über einen kleinen konischen Bereich 81 in eine sich bis zum zweiten Ende 7 hin erstreckende Bohrung 90.

Anstelle des kugelsegmentförmigen Abschnittes 10 in der zuvor beschriebenen Ausführungsform, der die Fassung für den Kopf 3 bildet, ist die Fassung für den Kopf 3 bei dieser Ausführungsform durch ein separates Einsatzteil 60 gebildet.

Das Einsatzteil 60 ist im wesentlichen hohlzylindrisch ausgebildet mit einem maximalen Außendurchmesser, der etwas kleiner als der Innendurchmesser der Bohrung 90 ist, so daß das Teil 60 in das Aufnahmeteil 90 von seinem zweiten Ende 7 her einschiebbar ist. Das Einsatzteil 60 weist ein erstes Ende auf mit einem an der Außenseite angefasten bzw. sich konisch verjüngenden Abschnitt 61, dessen Kegelwinkel dem Kegelwinkel des Abschnittes 81 des Aufnahmeteils 50 entspricht, so daß in eingesetztem Zustand der konische Abschnitt 61 des Einsatzteils 60 auf dem konischen Abschnitt 81 des Aufnahmeteils ruht.

An seiner Innenseite ist das Einsatzteil 60 angrenzend an den konischen Bereich 61 mit einem kugelsegmentförmigen Abschnitt 62 ähnlich dem Abschnitt 10 des Aufnahmeteils 5 der zuvor beschriebenen Ausführungsform ausgebildet, dessen Radius im wesentlichen dem Radius des Kopfes 3 entspricht. Der kugelsegmentförmige Abschnitt 62 bildet die Fassung für den Kopf 3. Ferner weist das Einsatzteil 60 an seinem dem konischen Abschnitt 61 gegenüberliegenden Ende zwei um 180° zueinander versetzte schlitzförmige Ausnehmungen 63 auf, die sich auf eine vorbestimmte Länge bis in den kugelsegmentförmigen Abschnitt 62 erstrecken. Die Breite und die Tiefe der Ausnehmungen 63 ist derart bemessen, daß die Ausnehmungen 63, wenn das Einsatzteil 60 in das Aufnahmeteil 50 eingesetzt ist, mit den schlitzförmigen Ausnehmungen 15 zur Deckung gelangen. Die Länge des Einsatzteiles 60 in axialer Richtung ist geringer als der Abstand des Grundes des Kanals vom ersten Ende 6 des Aufnahmeteils 50.

Zwischen dem Einsatzteil 60 und der Innenwand des Aufnahmeteils ist ferner eine in den Figuren nicht dargestellte Verdrehsicherung vorgesehen, damit die schlitzförmigen Ausnehmungen 15 des Aufnahmeteils und des Einsatzteils sich beim Einschrauben des Schraubenelements in den Knochen nicht gegeneinander verschieben. Eine derartige Verdrehsicherung kann beispielsweise durch umfangsseitig an dem Einsatzteil 60 vorgesehene Senkbohrungen, die mit entsprechenden Kröpfbohrungen am Aufnahmeteil zusammenwirken, realisiert sein.

Im Betrieb wird das Schraubenelement zuerst in das Einsatzteil 60 mit seinem Gewindeabschnitt 2 in Richtung des konischen Abschnitts 61 eingeführt bis der Kopf 3 in die Fassung 62 einschnappt, was aufgrund der durch die schlitzförmigen Ausnehmungen gegebenen Elastizität möglich ist. Dann wird das Einsatzteil 60 mitsamt dem Schraubenelement in das Aufnahmeteil 50 eingeführt bis der konische Abschnitt 61 des Einsatzteiles auf dem konischen Abschnitt 81 des Aufnahmeteils ruht. Das Schraubenelement wird anschließend in den Knochen eingeschraubt. Die Ausrichtung des Aufnahmeteils sowie die Fixierung von Kopf 3 und Stab 12 über die Mutter 17 erfolgt wie bei der ersten Ausführungsform. Die gegeneinander bewegten Schenkel 13, 14 des Aufnahmeteils üben bei dieser Ausführungsform über das geschlitzte Einsatzteil 60 eine Klemmkraft auf den Kopf aus.

In einer Abwandlung sind die schlitzförmigen Ausnehmungen 63 des Einsatzteiles tiefer als die Ausnehmungen 15 des Aufnahmeteils 50. Damit wird aufgrund der größeren Elastizität des Einsatzteiles das Einsetzen des Schraubenelements erleichtert, während das Aufnahmeteil stabil genug ausgelegt werden kann.

Die das Einsatzteil 60 beinhaltende Ausführungsform hat zum einen den Vorteil, daß das Aufnahmeteil 50 leichter zu fertigen ist, als bei der ersten Ausführungsform, zum anderen erleichtert das vorherige Einsetzen des Schraubenelements die Handhabung während der Anwendung.

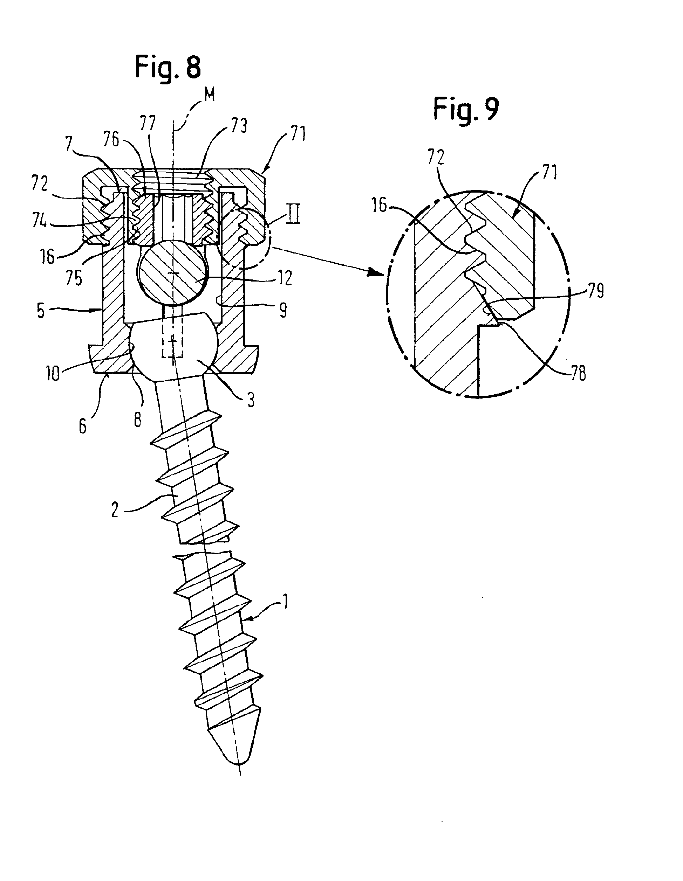

Die in den Figuren 8 und 9 dargestellte zweite Ausführungsform und deren Abwandlung unterscheidet sich von den in den Figuren 1 bis 7 dargestellten Ausführungsform durch eine getrennte Fixierung von Kopf 3 und Stab 12.

Bei der in Fig. 8 dargestellten Ausführungsform ist das Aufnahmeteil 5 wie das Aufnahmeteil gemäß den Figuren 1 bis 5 ausgebildet.

Anstelle der Mutter 17 ist eine Kappe 71 vorgesehen, die ein mit dem Außengewinde 16 des Aufnahmeteils 5 zusammenwirkendes Innengewinde 72 aufweist. Die Kappe 71 weist ferner eine zentrale Bohrung 73 und einen sich davon in Richtung des freien Randes erstreckenden zylindrischen Ansatz 74 mit einem Innengewinde 75 zur Aufnahme einer Stabfixierschraube 76 auf. Der zylindrische Ansatz 74 hat einen Durchmesser, der etwas geringer ist als der Innendurchmesser der Bohrung 9 des Aufnahmeteils, so daß beim Aufschrauben der Kappe der zylindrische Ansatz in das Aufnahmeteil gleitet und genügend Spiel zur Innenwand des Aufnahmeteils besteht, so daß ein Zusammendrücken der Schenkel zum Verklemmen des Kopfes wie bei der ersten Ausführungsform möglich ist. Der zylindrische Ansatzes 74 bildet ferner einen Anschlag für die Schenkel 13, 14 derart, daß die Schenkel nur soweit zusammengedrückt werden können, daß der Stab in dem Kanal noch frei verschiebbar ist. Die Stabfixierschraube 76 ist als Setzschraube ausgebildet und weist eine Ausnehmung 77 zum Ineingriffbringen mit einem Eindrehwerkzeug auf. In vollständig aufgeschraubtem Zustand der Kappe (in Fig. 8 nicht dargestellt) stößt die Kappe am freien Ende 7 der Schenkel 13, 14 an, wodurch ein Anschlag gebildet ist. Die Abmessungen der Kappe 71 und die Länge des Innengewindebereichs der Kappe sind so bemessen, daß in vollständig aufgeschraubtem Zustand die Kappe nicht auf den eingelegten Stab 12 drückt.

Im Betrieb wird das Schraubenelement in das Aufnahmeteil eingesetzt und in den Knochen eingeschraubt wie bei der ersten Ausführungsform. Anschließend wir der Stab eingelegt und die Kappe 71 mit eingeschraubter Stabfixierschraube 76 lose aufgeschraubt. Eine Justierung der Stellung des Aufnahmeteils und eine Justierung des Stabes sind in diesem Zustand noch möglich. Durch vollständiges Aufschrauben der Kappe bis zum Anschlag wird wie beim Aufschrauben der Mutter gemäß der ersten Ausführungsform ein Zusammendrücken der Schenkel des Aufnahmeteils bewirkt, was zu einem Verklemmen des Kopfes 3 in der Fassung führt, während der Stab in dem Kanal noch längsverschiebbar ist. Durch weiteres Einschrauben der Stabfixierschraube 76 bis deren Unterseite auf den Stab drückt wird dann der Stab fixiert.

In Fig. 9 ist eine Abwandlung dieser Ausführungsform gezeigt. Das Aufnahmeteil 5 ist wie zuvor beschrieben ausgebildet mit dem Unterschied daß an dem dem ersten Ende 6 des Aufnahmeteils zugewandten Ende des Außengewindes 16 in der Außenwand des Aufnahmeteils eine rundumlaufende schräge Fläche 78 vorgesehen ist, die unter einem Winkel von etwa 30° zur Mittenachse M des Aufnahmeteils in Richtung auf das erste Ende 6 hin ausgerichtet ist.

Angrenzend an den freien Rand der Kappe 71 ist entsprechend an der Innenseite eine schräge Fläche 79 vorgesehen, die in aufgeschraubtem Zustand der Kappe mit der schrägen Fläche 78 des Aufnahmeteils zusammenwirkt. Die Breite der schrägen Fläche 79 der Kappe ist etwas geringer als die Breite der schrägen Fläche 78 des Aufnahmeteils, so daß beim Aufschrauben der Kappe die Flächen in gewissem Umfang aufeinander gleiten können.

Im Betrieb gelangen bei dieser abgewandelten Ausführungsform beim Aufschrauben der Kappe bis sie am freien Ende der Schenkel anstößt, die schrägen Flächen 78 und 79 aufeinander, wobei eine zusätzliche Kraft auf die Schenkel in Richtung auf die Mittenachse M des das Aufnahmeteils ausgeübt wird, die die Schenkel zusammendrückt.

Bei der in den Figuren 8 und 9 dargestellten Ausführungsform ist es auch möglich das Einsatzteil 60 gemäß der in den Figuren 6 und 7 dargestellten Ausführungsform zu verwenden.

In den Figuren sind die schlitzförmigen Ausnehmungen 15 des Aufnahmeteils 5 bzw. 50 und des Einsatzteils 60 mit rechtekkigem Grund gezeigt. Sie können jedoch auch als Rundung ausgebildet sein.

Die Erfindung wurde anhand eines Beispiels einer Knochenschraube beschrieben. Anstelle der Knochenschraube kann auch Haken vorgesehen sein, wobei dieser dann zweiteilig ausgebildet ist und einen Kopf und einen damit verbindbaren Hakenabschnitt aufweist, der erst verbunden wird , wenn der Kopf eingesetzt ist.

Der Begriff Stab wird im vorhergehenden stellvertretend für alle stabförmigen Elementen verwendet und schließt diese mit ein.

## Patentansprüche

1. Aufnahmeteil zum gelenkigen Verbinden eines Knochenverankerungselements (1), welches einen Schaftteil (2) und einen Kopf (3) aufweist, mit einem Stab (12), der einen vorbestimmten Durchmesser aufweist, wobei das Aufnahmeteil aufweist:
ein erstes Ende (6) und ein diesem gegenüberliegendes zweites Ende (7),
eine sich von dem ersten Ende (6) in Richtung des zweiten Endes (7) erstreckende Bohrung (8) zum Hindurchführen des Schaftteils (2),
angrenzend an die Bohrung (8) einen Abschnitt (10; 60, 62) zur Aufnahme des Kopfes (3),
eine sich von dem zweiten Ende (7) in Richtung des ersten Endes (6) erstreckende Öffnung (9) zum Einführen des Schaftteils mit Kopf,
eine sich von dem zweiten Ende (7) in Richtung des ersten Endes (6) erstreckenden Ausnehmung (11) zum Bilden eines Kanals (11b) zum Einlegen des Stabs wodurch zwei freie Schenkel (13, 14) gebildet sind, und
zwei sich stirnseitig vom Grund des Kanals (11b) in Richtung des ersten Endes (6) erstreckende schlitzförmige Ausnehmungen (15);
**dadurch gekennzeichnet, dass** die Breite des Kanals (11b) bei eingelegtem Stab (12) im unbelasteten Zustand größer ist, als der vorbestimmte Durchmesser des Stabs, und wobei das Aufnahmeteil so ausgebildet ist, dass der Kopf (3) durch Zusammendrücken der Schenkel (13, 14) blockiert wird.

2. Knochenverankerungsvorrichtung zum Stabilisieren von Knochensegmenten mit
einem Knochenverankerungselement (1), welches einen Schaftteil (2) und einen Kopf (3) aufweise,
einem Aufnahmeteil (5; 50) nach Anspruch 1 zum gelenkigen Verbinden des Knochenverankerungselements (1) mit einem Stab (12),

3. Element nach Anspruch 1 oder 2, **gekennzeichnet durch** eine Einrichtung (16, 17; 16, 71, 72; 16, 71, 72, 78, 79) zum Ausüben einer Kraft auf die freien Schenkel zum Gegeneinanderdrücken der Schenkel.

4. Element nach einem der Ansprüche 1 bis 3, **dadurch gekennzeichnet, dass** der Abschnitt (10; 60, 62) zur Aufnahme des Kopfes (3) eine dem aufzunehmenden Kopf entsprechende Form aufweist.

5. Element nach einem der Ansprüche 1 bis 4, **dadurch gekennzeichnet, dass** der Kopf (3) kugelsegmentförmig ausgebildet ist und der Abschnitt (10; 62) zur Aufnahme des Kopfes eine entsprechende kugelsegmentförmige Fassung für den Kopf aufweist.

6. Element nach einem der Ansprüche 1 bis 5, **dadurch gekennzeichnet, dass** ein in dem Aufnahmeteil (5; 50) vorgesehenes separates Einsatzteil (60) zum Fassen des Kopfes vorgesehen ist, wobei das Einsatzteil entsprechende schlitzförmige Ausnehmungen (63) aufweist, die mit den schlitzförmigen Ausnehmungen (15) in eingesetztem Zustand zu Deckung gelangen.

7. Element nach einem der Ansprüche 3 bis 6, **dadurch gekennzeichnet, dass** die Einrichtung (16, 17; 16, 71, 72; 16, 71, 72, 78, 79) zum Ausüben einer Kraft auf die freien Schenkel ein Außengewinde (16) an den freien Schenkeln und eine Mutter (17; 71) mit einem mit dem Außengewinde zusammenwirkenden Innengewinde umfasst.

8. Element nach Anspruch 7, **dadurch gekennzeichnet, dass** die Abmessungen des Außengewindes (16) und der Mutter (17) so gewählt sind, dass die Mutter in soweit aufschraubbar ist, dass sie auf den eingelegten Stab drückt.

9. Element nach Anspruch 7, **dadurch gekennzeichnet, dass** die Mutter (71) derart ausgebildet ist, dass sie in vollständig aufgeschraubtem Zustand den Stab nicht fixiert und dass eine Stabfixierschraube (76) zum Fixieren des Stabes vorgesehen ist.

10. Element nach Anspruch 9, **dadurch gekennzeichnet, dass** das Außengewinde (16) der Schenkel und das Gewinde (72) der Mut-ter (71) jeweils einen Abschnitt (78, 79) aufweisen, über dem beim Zusammenwirken eine Druckkraft auf die freien Schenkel in einer Richtung senkrecht zur schlitzförmigen Ausnehmung ausgeübt wird.

11. Element nach Anspruch 9 oder 10, **dadurch gekennzeichnet, dass** die Mutter (71) kappenförmig ausgebildet ist mit einer zentralen Bohrung (73) mit einem zylindrischen Ansatz (74) mit Innengewinde (75) zur Aufnahme der Stabfixierschraube (76).

## Claims

1. Receiver part for articulatedly connecting a bone anchoring element (1), comprising a shank part (2) and a head (3), with a rod (12) having a predetermined diameter, wherein the receiver part comprises:
a first end (6) and a second end (7) opposite thereto;
a bore (8) extending from the first end (6) in the direction of the second end (7) for guiding-through the shank part (2) ;
adjacent to the bore (8), a section (10; 60, 62) for receiving the head (3);
an opening (9) extending from the second end (7) in the direction of the first end (6) for inserting the shank part with head;
a recess (11) extending from the second end (7) in the direction of the first end (6) for forming a channel (11b) for inserting the rod, whereby two open legs (13, 14) are formed; and
two slit-shaped recesses (15) extending at the front side from the bottom of the channel (11b) in the direction of the first end (6),
**characterized in that** the width of the channel (11b) with the rod (12) inserted is in the unloaded state larger than the predetermined diameter of the rod and
wherein the receiver part is formed such that the head (3) is locked by pressing the legs (13, 14) together.

2. Bone anchoring device for stabilizing bone segments with
a bone anchoring element (1) comprising a shank part (2) and a head (3);
a receiver part (5; 50) according to claim 1 for articulatedly connecting the bone anchoring element (1) to a rod (12).

3. Element according to claim 1 or 2, **characterized by** a device (16, 17; 16, 71, 72; 16, 71, 72, 78, 79) for exerting a force on the open legs for pressing the legs together.

4. Element according to one of claims 1 to 3, **characterized in that** the section (10; 60, 62) for receiving the head (3) has a shape corresponding to the head to be received.

5. Element according to one of claims 1 to 4, **characterized in that** the head (3) is formed with a shape of a segment of a sphere and the section (10; 62) for receiving the head has a corresponding holder shaped as a segment of a sphere for the head.

6. Element according to one of claims 1 to 5, **characterized in that** a separate insertion part (60) provided in the receiver part (5; 50) for holding the head is provided, wherein the insertion part comprises corresponding slit-shaped recesses (63) that come to overlap with said slit-shaped recesses (15) in the inserted state.

7. Element according to one of claims 3 to 6, **characterized in that** the device (16, 17; 16, 71, 72; 16, 71, 72, 78, 79) for exerting a force on the open legs contains an outer thread (16) on the open legs and a nut (17; 71) with an inner thread that cooperates with the outer thread.

8. Element according to claim 7, **characterized in that** the dimensions of the outer thread (16) and of the nut (17) are chosen such that the nut can be screwed down to such an extent that it presses on the inserted rod.

9. Element according to claim 7, **characterized in that** the nut (71) is formed such that, in the fully screwed down state, it does not fix the rod and **in that** a rod fixing screw (76) for fixing the rod is provided.

10. Element according to claim 9, **characterized in that** the outer thread (16) of the open legs and the thread (72) of the nut (71) each comprise a section (78, 79) by which, when cooperating, a pressure force is exerted on the open legs in a direction perpendicular to the slit-shaped recess.

11. Element according to claim 9 or 10, **characterized in that** the nut (71) is formed cap-shaped with a central bore (73) and with a cylindrical projection (74) with an inner thread (75) for receiving the rod fixing screw (76).

## Revendications

1. Pièce de réception pour la liaison articulée d'un élément d'ancrage osseux (1), comportant une partie tige (2) et une tête (3), avec une barre (12) qui a un diamètre prédéfini, la pièce de réception comportant :
une première extrémité (6) et une deuxième extrémité (7) opposée à cette dernière,
un alésage (8) s'étendant de la première extrémité (6) en direction de la deuxième extrémité (7), pour y faire passer la partie tige (2),
une section (10 ; 60, 62) adjacente à l'alésage (8), pour la réception de la tête (3),
un orifice (9) s'étendant de la deuxième extrémité (7) en direction de la première extrémité (6), pour y introduire la partie tige avec la tête,
un évidement (11) s'étendant de la deuxième extrémité (7) en direction de la première extrémité (6), pour former un conduit (11b) pour y insérer la barre, ce qui a pour effet de former deux branches libres (13, 14), et
deux évidements (15) en forme de fentes, s'étendant sur la face frontale du fond du conduit (11b), en direction de la première extrémité (6) ;
**caractérisée en ce que** lorsque la barre (12) est insérée et à l'état non chargé, la largeur du conduit (11b) est supérieure au diamètre prédéfini de la barre, et la pièce de réception étant conçue de façon telle, que la tête (3) soit bloquée par compression des branches (13, 14).

2. Dispositif d'ancrage osseux, pour stabiliser deux segments osseux, avec
un élément d'ancrage osseux (1) comportant une partie tige (2) et une tête (3),
une pièce de réception (5 ; 50) selon la revendication 1, pour la liaison articulée de l'élément d'ancrage osseux (1) avec une barre (12).

3. Elément selon la revendication 1 ou 2, **caractérisé par** un dispositif (16, 17 ; 16, 71, 72 ; 16, 71, 72, 78, 79) pour exercer une force sur les branches libres, pour presser les branches l'une contre l'autre.

4. Elément selon l'une quelconque des revendications 1 à 3, **caractérisé en ce que** la section (10 ; 60, 62) pour la réception de la tête (3) présente une forme correspondant à la tête devant être réceptionnée.

5. Elément selon l'une quelconque des revendications 1 à 4, **caractérisé en ce que** la tête est conçue sous la forme d'un segment sphérique et **en ce que** la section (10 ; 62) pour la réception de la tête (3) comporte une prise correspondante en forme de segment sphérique pour la tête.

6. Elément selon l'une quelconque des revendications 1 à 5, **caractérisé en ce qu'**une pièce d'insertion (60) séparée prévue dans la pièce de réception (5 ; 50) est prévue pour la prise de la tête, la pièce d'insertion comportant des évidements (63) correspondants en forme de fentes, qui lorsqu'ils sont insérés se juxtaposent aux évidements (15) en forme de fentes.

7. Elément selon l'une quelconque des revendications 3 à 6, **caractérisé en ce que** le dispositif (16, 17 ; 16, 71, 72 ; 16, 71, 72, 78, 79) pour exercer une force sur les branches libres comporte un filetage (16) sur les branches libres et un écrou (17 ; 71) avec un taraudage, qui est en interaction avec le filetage.

8. Elément selon la revendication 7, **caractérisé en ce que** les dimensions du filetage (16) et de l'écrou (17) sont choisies de façon à permettre de visser l'écrou assez loin, pour qu'il exerce une pression sur la barre insérée.

9. Elément selon la revendication 7, **caractérisé en ce que** l'écrou (71) est conçu de façon à ce qu'à l'état entièrement vissé, il ne fixe pas la barre et **en ce qu'**une vis de fixation de la barre (76) est prévue pour fixer la barre.

10. Elément selon la revendication 9, **caractérisé en ce que** le filetage (16) des branches et le taraudage (72) de l'écrou (71) comportent respectivement une section (78, 79) sur laquelle lors de leur interaction, une force de pression est exercée sur les branches libres, dans une direction perpendiculaire à l'évidement en forme de fente.

11. Elément selon la revendication 9 ou 10, **caractérisé en ce que** l'écrou (71) est conçu sous la forme d'une calotte, avec un alésage central (73) comportant un embout cylindrique (74) avec taraudage (75) pour la réception de la vis de fixation de la barre (76).
